# EUROPÄISCHE PATENTSCHRIFT

(11) **EP 1 270 412 B1**
(45) Veröffentlichungstag und Bekanntmachung des Hinweises auf die Patenterteilung: **14.09.2005**
(21) Anmeldenummer: 02012678.5
(22) Anmeldetag: 07.06.2002
(51) Int. Cl.: B65B 3/00, A61J 1/06

(54) **Verfahren zum Befüllen von im wesentlichen zylinderförmigen Ampullen**
Method for filling cylindrical ampoules
Procédé de remplissage pour ampoules cylindiques

(30) Priorität: 19.06.2001 DE 10129452
(43) Veröffentlichungstag der Anmeldung: 02.01.2003
(73) Patentinhaber: ROBERT BOSCH GMBH, 70442 Stuttgart (DE)
(72) Erfinder: Mayer, Werner, 74599 Wallhausen (DE); Klingler, Dieter, 74589 Satteldorf (DE)

(56) Entgegenhaltungen:
- WO-A-97/29954
- DE-C- 585 441
- DE-C- 19 647 694

## Beschreibung

Die Erfindung betrifft ein Verfahren zum Befüllen von im wesentlichen zylinderförmigen Ampullen nach dem Oberbegriff der unabhängigen Ansprüche 1, 4, 5, 6 und 7. Bei einem derartigen, aus der DE 585 441 C bekannten Verfahren wird ein Gummistopfen, welcher in das eine Ende einer Ampulle eingesetzt ist, mittels einer Kanüle durchstochen. Anschließend wird der in dem anderen Ende der Ampulle eingesetzte Stopfen in Richtung der Kanüle verschoben, so dass die im Kopfraum zwischen dem Füllgutpegel und dem Gummistopfen befindliche Luft durch die Kanüle entweichen kann. Zuletzt wird die Kanüle aus dem Gummistopfen herausgezogen, wodurch sich die Öffnung für die Kanüle verschließt. Ein derartiges Verfahren ist nur bei sich selbst abdichtenden Gummistopfen anwendbar.

Weiterhin ist es aus der WO 97/29954 bekannt, den oberen Bereich einer Ampulle mittels einer optischen Überwachungseinrichtung zu kontrollieren. Um eine Überfüllung, d.h. ein Überlaufen der Ampulle zu vermeiden, wird das Ventil der Fülleinrichtung geschlossen, sobald der Füllpegel das obere Ende der Ampulle erreicht hat. Mit dem bekannten Verfahren lassen sich zwar Luftblasen in den verschlossenen Ampullen vermeiden, jedoch ist die Füllgenauigkeit unbefriedigend. Dies ergibt sich aus der Ungenauigkeit bezüglich der Position des die untere Öffnung der Ampulle verschließenden Stopfens sowie aus den Geometrietoleranzen der Ampulle selbst. Mit anderen Worten gesagt bedeutet dies, daß abhängig von der Position des Stopfens und der Toleranz der Ampulle unterschiedliche Füllmengen eindosiert werden. Wenn aus gesetzlichen Vorschriften bezüglich der Füllmenge eine gewisse Mindestmenge gefordert ist, wird es daher im Mittel in der Massenfertigung bzw. Massenabfüllung stets zu einer gewissen Überdosierung kommen, um die geforderte Mindestmenge sicher einhalten zu können. Derartige Überdosierungen können jedoch, insbesondere bei der Abfüllung von relativ teuren Füllgütern, die Herstell- bzw. Abfüllerkosten nicht unerheblich erhöhen.

Aufgabe der Erfindung ist es daher, das aus der DE 585 441 C bekannte Verfahren derart weiterzubilden, dass auch für nicht aus Gummi bestehende Verschlußstopfen Lufteinschlüsse im Kopfraum minimiert bzw. vermieden werden, wobei gleichzeitig stets nur die erforderliche Mindestmenge in die Ampulle einzudosiert werden soll. Diese Aufgabe wird mit den Verfahren der unabhängigen Ansprüche 1, 4, 5, 6 und 7 gelöst.

Vorteilhafte Weiterbildungen des erfindungsgemäßen Verfahrens zum Befüllen von im wesentlichen zylinderförmigen Ampullen sind in den Unteransprüchen angegeben.

### Zeichnung

Ausführungsbeispiele der Erfindung sind in der Zeichnung dargestellt und werden nachfolgend näher erläutert. Es zeigen:
Figuren 1a bis 1d eine erste Anordnung zur Verdeutlichung des erfindungsgemäßen Verfahrens,
Figur 2 eine Darstellung des füllgutfreien Kopfraumvolumens über der Anzahl der Kompressionsvorgänge,
Figur 3 eine Darstellung des Verschiebeweges eines Stößels einer Ampulle bei verschiedenen Behandlungsschritten,
Figuren 4 und 5 eine zweite Anordnung zur Verdeutlichung des Verfahrens,
Figuren 6 und 7 eine dritte Anordnung zur Verdeutlichung des Verfahrens und
Figur 8 eine vierte Anordnung zur Verdeutlichung des Verfahrens

### Beschreibung der Ausführungsbeispiele

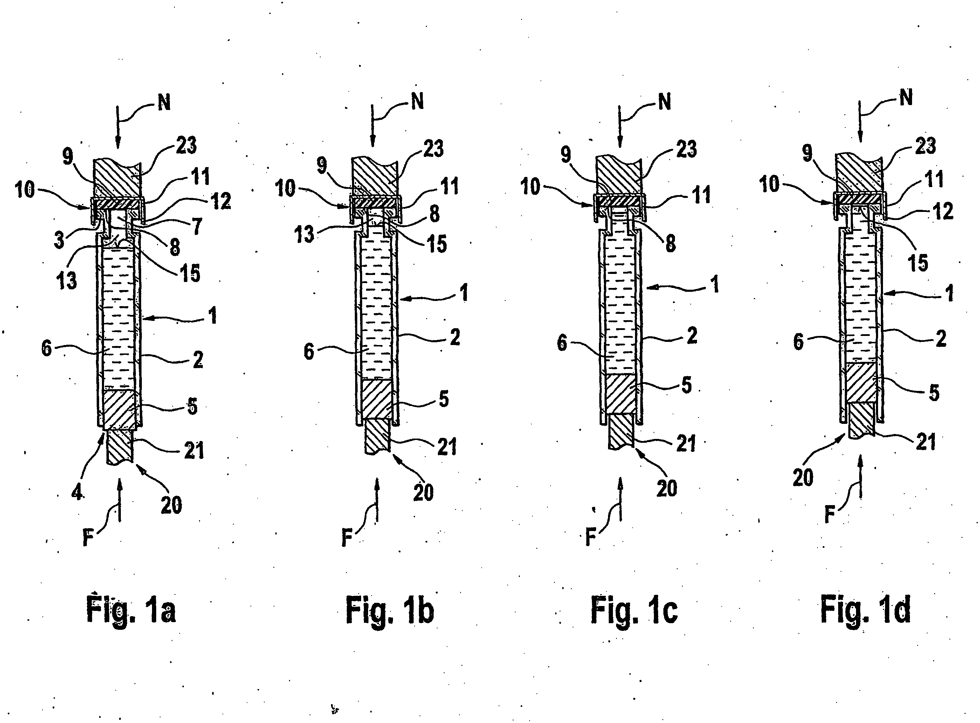

In den Figuren 1a bis 1d ist jeweils ein und dieselbe Ampulle 1 dargestellt, die einen zylinderförmigen Abschnitt 2 und einen kragenförmigen Halsbereich 3 aufweist. Das untere Ende des Abschnitts 2 weist eine erste Öffnung 4 auf, in die ein kolbenartiger Stopfen 5 teilweise eingeschoben ist. Mittels eines von einer Eindrückeinrichtung 20 in Richtung der Längsachse der Ampulle 1 nach oben verschiebbaren Stößels 21 läßt sich der Stopfen 5 weiter in die erste Öffnung 4 in Richtung des Halsbereiches 3 der Ampulle 1 eindrücken. Dabei ist zwischen der Innenwandung der Ampulle 1 und dem äußeren Umfang des Stopfens 5 ein Dichtsitz vorhanden, der die Ampulle 1 nach außen hin abdichtet.

Das Befüllen der Ampulle 1 mit einem flüssigen Füllgut 6, insbesondere einem Pharmazeutika, erfolgt über eine obere Öffnung 7 der Ampulle 1 vorab auf einer nicht dargestellten Fülleinrichtung, wobei das in der Ampulle 1 eindosierte Füllvolumen V möglichst exakt der Soll-Füllmenge der Ampulle 1 entspricht. Auf dem oberen Mündungsrand 8 der Ampulle 1 ist ein Dichtelement 9 angeordnet, das Teil eines Verschlusses 10 ist, der neben dem Dichtelement 9 eine aus Metall bestehende Verschlußkappe 11 aufweist. Der über den kragenförmigen Rand des Halsbereichs 3 der Ampulle 1 nach unten ragende Abschnitt 12 der Abschlußkappe 11 läßt sich zum luftdichten Verschließen der Ampulle 1 mittels einer aus dem Stand der Technik bekannten Bördeleinrichtung gegen den Halsbereich 3 umbördeln.

Von oben ist auf die Verschlußkappe 11 ein Niederhalter 23 aufsetzbar, der mit einer Kraft N belastbar ist, die ein Entweichen der Luft aus dem füllgutfreien Kopfraum 13 der Ampulle 1 verhindert, solange der Luftdruck in dem Kopfraum 13 einen vorgegebenen Maximalwert nicht überschreitet.

Der Stößel 21 der Eindrückeinrichtung 20 ist in Richtung des Verschlusses 10 mit einer vorzugsweise konstanten Kraft F belastbar, die in jedem Fall geringer ist als die entgegengesetze Kraft N des Niederhalters 23, jedoch eine Verschiebung des Stopfens 5 in Richtung des Verschlusses 10 bewirkt. Weiterhin ist die Kraft F derart begrenzt, daß eine Beschädigung der Ampulle 1 aufgrund der Kompression des Füllgutes 6 bzw. der Luft im füllgutfreien Kopfraum 13 ausgeschlossen ist.

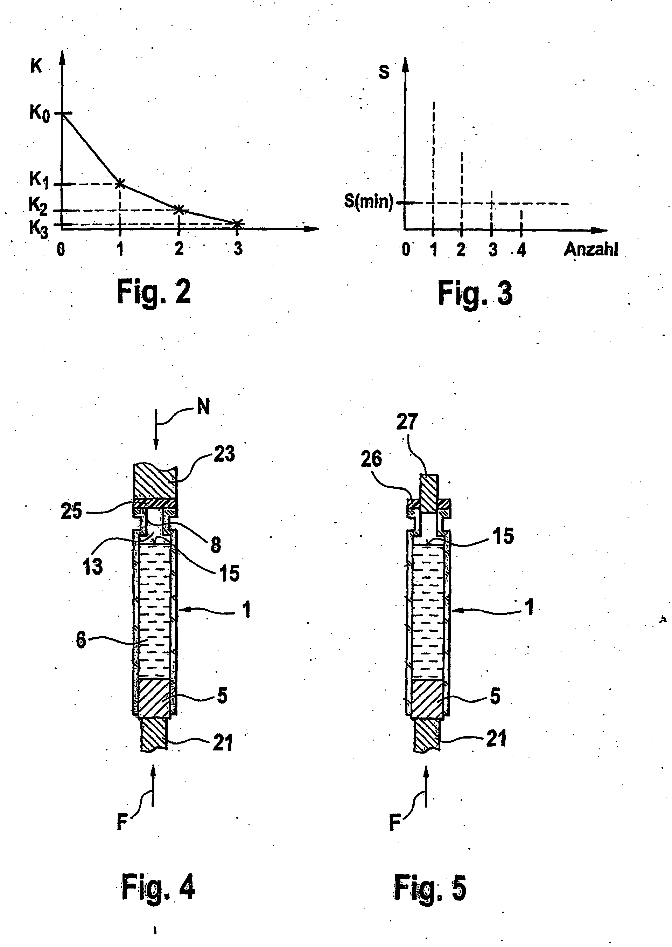

Erfindungswesentlich ist ein Verfahren, das dazu dient, das Volumen K des füllgutfreien Kopfraums 13 zu minimieren, so daß bei verschlossener Ampulle 1 Lufteinschlüsse in der Ampulle 1 vermieden werden. Mit Bezug auf die Figuren 1 und 2 läßt sich das Verfahren wie folgt erläutern: Bei der Figur 1a ist die Ampulle 1 mit dem Füllvolumen V, das der Sollfüllmenge entspricht, befüllt worden. Dabei befindet sich der Stopfen 5 zunächst in einer unteren Position, in der der Füllpegel 15 des Füllguts 6 unter Berücksichtigung des Füllvolumens V unterhalb des Mündungsrandes 8 der Ampulle 1 verläuft. Diesem Zustand ist das Kopfraumvolumen K₀ zugeordnet.

Anschließend wird zunächst der Niederhalter 23 und anschließend der Stößel 21 in ihre jeweilige Richtung über eine (nicht dargestellte) Steuereinrichtung mit ihrer jeweiligen Kraft N und F beaufschlagt. Dadurch wird der Stopfen 5 in Richtung des Mündungsrandes 8 bewegt, wobei sich gleichzeitig das Kopfraumvolumen K durch Kompression der im Kopfraum 13 eingeschlossenen Luft auf den Wert K₁ verringert. Dann werden die Kräfte F und N derart reduziert, daß die unter Überdruck, weil komprimierte Luft aus dem Kopfraum 13 über den entlasteten Kopfverschluß 10 bzw. das Dichtelement 9 aus der Ampulle 1 entweichen kann.

Anschließend wiederholen sich die Vorgänge wie eben beschrieben. So erkennt man an den Figuren 1c und 1d, denen die beiden nachfolgenden Verdichtungsvorgänge zugeordnet sind, daß sich der Füllpegel 15 mehr und mehr dem Mündungsrand 8 der Ampulle 1 nähert, während gleichzeitig das Kopfraumvolumen K über den Wert K₂ auf den Wert K₃ reduziert wird. Am Kurvenverlauf der Figur 2 erkennt man, daß mit dem vorgeschlagenen Verfahren das füllgutfreie Kopfraumvolumen K sich asymptotisch dem Wert Null nähert, während gleichzeitig entsprechend den Figuren 1 der Füllpegel 15 sich nach und nach dem Mündungsrand 8 annähert.

Die Anzahl der erforderlichen Kompressionsvorgänge, die im dargestellten Ausführungsbeispiel drei beträgt, ist abhängig von dem füllgutfreien Kopfvolumen K zu Beginn, d.h. der ursprünglichen Position des Stopfens 5 in Bezug zum Mündungsrand 8, sowie insbesondere auch mit der Höhe der Kraft F, mit der der Stopfen 5 in Richtung des Mündungsrandes 8 gedrückt wird, da davon die Kompression der im Kopfraum 13 der Ampulle 1 befindlichen Restluft abhängt.

In der Praxis wird man die Anzahl der erforderlichen Kompressionsvorgänge anhand von Versuchsreihen ermitteln und als Festwert der Steuereinrichtung, die den Stößel 21 und den Niederhalter 23 ansteuert, vorgeben. Dadurch, daß die Kraft N stets größer ist als die Kraft F, ist ein Entweichen von Flüssigkeit aus der Ampulle 1, genügend Dichtwirkung des Dichtelements 9 vorausgesetzt, ausgeschlossen. Nach einer entsprechenden Anzahl an Kompressionsvorgängen wird somit der Füllpegel 15 des Füllgutes 6 mit dem Mündungsrand 8 derart annähern, daß er meßtechnisch mit diesem zusammenfällt.

Bei einem abgewandelten Verfahren ist es möglich, anstelle einer vorgegebenen Anzahl an Kompressionsvorgängen den Verschiebeweg s des Stopfens 5 mittels einer Meßeinrichtung zu erfassen, wenn der Stopfen 5 mit einer vorgegebenen und vorzugsweise konstanten Kraft F in Richtung des Mündungsrandes 8 belastet wird. Da der Verschiebeweg s bei jedem Kompressionsvorgang infolge des immer geringer werdenden Kopfraumvolumens K ebenfalls immer geringer wird, werden sich die erfassten Verschiebwege s einstellen, wie sie in der Figur 3 qualitativ dargestellt ist. Da ein mathematischer Zusammenhang zwischen dem Kopfraumvolumen K und dem Verschiebeweg s besteht, ist es nunmehr vorgesehen, die Kompressionsvorgänge zu beenden, sobald der Verschiebeweg s bei einem Kompressionsvorgang unter einen vorgebenen Grenzwert s(min) fällt, der auf ein bestimmtes Kopfraumvolumen K schließen läßt. Im dargestellten Fall entsprechend der Figur 3 wird somit das Verschieben des Stopfens 5 in Richtung des Mündungsrandes 8 nach dem vierten Kompressionsvorgang beendet.

Bei einem abgewandelten erfindungsgemäßen Verfahren entsprechend den Figuren 4 und 5 wird im Gegensatz zu dem ersten erfindungsgemäßen Verfahren der Stopfen 5 bereits bei einem zweiten Verschieben des Stopfens 5 der Füllpegel 15 in Deckung mit dem Mündungsrand 8 der Ampulle 1 gebracht, so daß das Kopfraumvolumen K minimiert ist. Dies erfolgt dadurch, daß zuerst der Mündungsrand 8 der Ampulle 1 mittels des Dichtelements 25, welches mit dem mit der Kraft N beaufschlagten Niederhalter 23 verbunden ist, dicht verschlossen wird. Dann wird über den Stößel 21 eine Kraft F(ref) auf den Stopfen 5 ausgeübt, die diesen in die Ampulle 1 um einen bestimmten, vorgegebenen Weg s(ref) weiter eindrückt. Wesentlich dabei ist nun, daß sowohl der Verschiebeweg s, als auch der Kraftverlauf F des Stopfens 5 erfasst werden. Aufgrund der physikalischen Eigenschafetn der Luft sowie der bekannten Geometrie der Ampulle 1 und des Stopfens 5 mit den bekannten Reibverhältnissen zwischen Stopfen 5 und Ampulle 1 läßt sich nunmehr aufgrund der erforderlichen Kraft F(ref), die zum Verschieben des Stopfens 5 um den Weg s(ref) geführt hat, ein ursprüngliches bzw. ein verbleibendes Kopfraumvolumen K berechnen.

Sobald der Stopfen 5 um den Weg s(ref) verschoben wurde, wird zuerst die Kraft F und dann die Kraft N reduziert, um die komprimierte Luft aus dem Kopfraum 13 entweichen lassen zu können. Es ist jedoch auch möglich, den Stößel 21 ortsfest zu belassen und statt dessen, die Kraft N zu reduzieren, wodurch sich auch die Kraft F entspannt bzw. reduziert.

Aufgrund des errechneten Kopfraumvolumens K kann nun der Stopfen 5 um einen daraus resultierenden Verschiebeweg s, der den Füllpegel 15 bis in Höhe des Mündungsrandes 8 anhebt, in die Ampulle 1 weiter eingedrückt werden, wobei während dieses Vorgangs die Kraft N des Niederhalters 23 reduziert ist, damit die im Kopfraum 13 befindliche Luft gleichzeitig entweichen kann.

Bei der Anordnung gemäß der Figur 5 wird ebenfalls prizipiell das zuletzt beschriebene erfindungsgemäße Verfahren angewandt. Im Unterschied dazu wird jedoch ein im Dichtelement 26 angeordneter Drucksensor 27 verwendet, der den Druckverlauf im Kopfraum 13 während des Verschiebens um den Weg s(ref) des Stopfens 5 direkt erfasst. Es ist somit keine Erfassung der erforderlichen Kraft F für den Stopfen 5 erforderlich. Hiermit lassen sich im Gegensatz zu der Anordnung nach Figur 4, bei der aus der erforderlichen Kraft F auf den Druck im Kopfraum 13 geschlossen wird, Unterschiede in den Reibungsverhältnissen zwischen Stopfen 5 und Ampulle 1 vernachlässigen.

Zusätzlich wird erwähnt, daß sich die beiden bisher beschriebenen erfindungsgemäßen Verfahren auch kombinieren lassen. Hierbei ist insbesondere daran gedacht, in einem ersten Schritt zuerst das zweite Verfahren anzuwenden, wobei der Stopfen 5 bis knapp unterhalb des Mündungsrandes 8 verschoben wird, und anschließend einen oder mehrere Schritte des ersten Verfahrens folgen zu lassen. Dieses kombinierte Verfahren ist insbesondere dann vorteilhaft, wenn die Druckerfassung des Kopfraumes 13 über den Kraftverlauf F erfasst wird, da dieser aufgrund der Toleranzen in Bezug auf die Reibverhältnisse zwischen Stopfen 5 und Ampulle 1 ungenauer ist als die direkte Erfassung über den Drucksensor 27. Weiterhin können so Toleranzen der Ampulle 1 besser ausgeglichen werden. Ein derartiges Vorgehen hätte außerdem den Vorteil, daß es infolge des fehlenden Drucksensors 27 vorrichtungstechnisch einfacher ist, und daß anstelle des Dichtelements 26 gleich der spätere Verschluß 10 als Abdichtung verwandt werden kann (wie dies im Übrigen auch gemäß der Anordnung nach Figur 4 möglich ist).

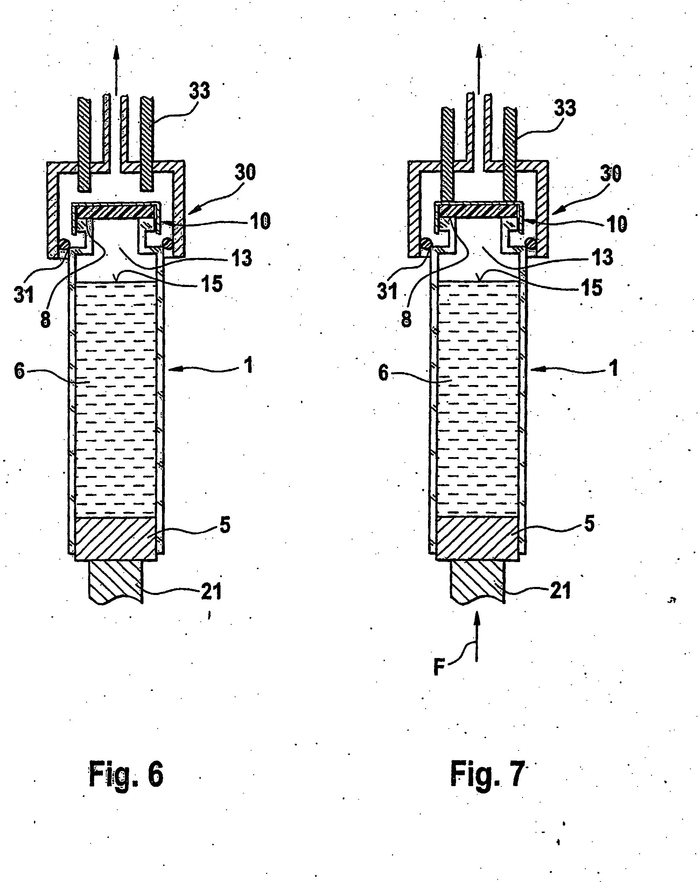

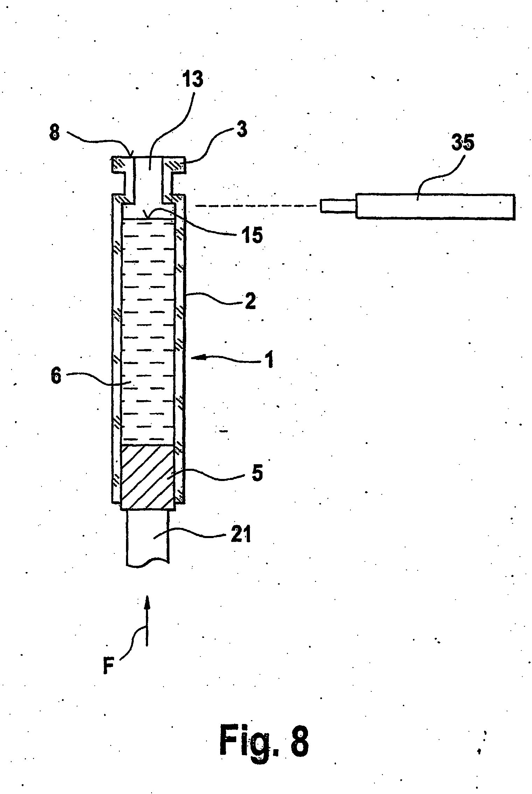

Bei einer weiteren Abwandlung des erfindungsgemäßen Verfahrens gemäß den Figuren 6 und 7 wird eine Vakuumglocke 30 verwendet, die an der Schulter 31 der Ampulle 1 dichtend angelegt werden kann. Die Vakuumglocke 30 ist von einem rohrartigen Niederhalter 33 durchdrungen, der in Anlagekontakt mit dem Verschluß 10 der Ampulle 1 gebracht werden kann. Bei diesem Verfahren erfolgt in einem ersten Schritt das Evakuieren des Kopfraumes 13 über die Vakuumglocke 30, wobei der Niederhalter 33 den Verschluß 10 nicht bzw. nur mit einer derart geringen Kraft auf den Mündungsrand 8 drückt, daß die Luft aus dem Kopfraum 13 evakuiert werden kann (Figur 6). In einem zweiten Schritt wird nun der Niederhalter 33 auf den Verschluß 10 abgesenkt, so daß die Ampulle 1 am Mündungsrand 8 abgedichtet ist. Jetzt wird der Stopfen 5 mittels des Stößels 21 in Richtung des Mündungsrandes 8 verschoben, wobei gleichzeitig die während des Verschiebens vom Stopfen 5 auf den Stößel 21 einwirkende Reaktionskraft F(reak) erfasst wird (Figur 7).

Sobald der Stopfen 5 soweit in die Ampulle 1 eingedrückt ist, daß der Füllpegel 13 den Mündungsrand 8 bzw. den Verschluß 10 erreicht, wird die Reaktionskraft F(reak) infolge des inkompressiblen Füllguts 6 sprunghaft ansteigen, was für die die Stopfenbewegung steuernde Steuereinrichtung als Kriterium bewertet wird, das Einschieben des Stopfens 5 zu beenden.

Ergänzend wird erwähnt, daß es bei dem zuletzt beschriebenen erfindungsgemäßen Verfahren zur Vermeidung der Bildung von Gasblasen im Bereich des Stopfens 5 erforderlich sein kann, bereits die Füllung der Ampulle 1 mit dem Füllgut 6 unter Vakuum erfolgen zu lassen. Ferner kann das Erreichen des Mündungsrandes 8 des Füllpegels 13 auch mit Hilfe eines gefedert angeordneten Stößels 21 erkannt werden, der in diesem Fall aufgrund der sprunghaft ansteigenden Reaktionskraft F(reak) in diesem Moment stark einfedert.

Bei der zuletzt beschriebenen Abwandlung des erfindungsgemäßens Verfahren gemäß der Figur 8 wird eine optische Kontrolleinrichtung 35 verwendet, die derart positioniert ist, daß sie die Ampulle 1 vorzugsweise im Bereich ihres zylindrischen Abschnitts 2 nahe des Halsbereichs 3 erfasst, da dieser Bereich leichter erfassbar ist als der Halsbereich 3 selbst. Wesentlich ist auch, daß der Füllpegel 13 bzw. die Position des Stopfens 5 nach dem Befüllen der Ampulle 1 derart abgestimmt sind, daß der Füllpegel 13 zunächst unterhalb des von der Kontrolleinrichtung 35 erfassten Bereichs an der Ampulle 1 ist. Ferner ist wesentlich, daß aufgrund der Positionierung der Ampulle 1 zur Kontrolleinrichtung 35 der Abstand zwischen dem von der Kontrolleinrichtung 35 erfassten Bereich und dem Mündungsrand 8 der Steuereinrichtung bekannt ist. Das erfindungsgemäße Verfahren wird derart durchgeführt, daß der Stopfen 5 von dem Stößel 21 in Richtung des Mündungsrandes 8 in die Ampulle 1 eingedrückt wird. Sobald der Füllpegel 13 den Bereich erreicht, der von der Kontrolleinrichtung 35 erfasst wird, wird der Stopfen 5 um den bekannten Weg in die Ampulle 1 weiter eingedrückt, bis der Füllpegel 13 mit dem Mündungsrand 8 zusammenfällt.

Ergänzend wird erwähnt, daß bei den Ausführungsformen entsprechend der Figuren 1, 4, 6 und 7 auch spezielle Abdichtelemente für den Mündungsrand 8 verwendet werden können, die im Nachhinein durch die Verschlüsse 10 ersetzt werden.

Weiterhin wurde bei allen Ausführungsbeispielen aus Vereinfachungsgründen auf die Darstellung von Halterungen für die Ampullen 1 verzichtet. Selbstverständlich sind diese jedoch vorhanden.

## Patentansprüche

1. Verfahren zum Befüllen von im wesentlichen zylinderförmigen Ampullen (1), welche zwei Öffnungen (4, 7) aufweisen, eine untere Öffnung (4), in der ein kolbenartiger Stopfen (5) verschiebbar angeordnet ist, und eine obere Einfüllöffnung (7) für ein flüssiges Füllgut (6), die von einer Abdeckkappe (11), gegebenenfalls unter Zwischenlage eines Dichtelements (9), nach dem Befüllen verschlossen wird, wobei der Füllpegel (15) des Füllgutes (6) zur Vermeidung von Einschluss von Luft in den Ampullen (1) bis in Höhe des Mündungsrandes (8) der oberen Einfüllöffnung (7) ragt, wobei der Stopfen (5) zunächst in einer Position angeordnet ist, in der der Füllpegel (15) des Füllgutes (6) unter Berücksichtigung einer Soll-Füllmenge (V) unterhalb des Mündungsrandes (8) der Einfüllöffnung (7) verläuft, wobei anschließend die Soll-Füllmenge (V) in die jeweilige Ampulle (1) eingebracht wird und wobei zuletzt der Stopfen (5) in eine Position verschoben wird, in der der Füllpegel (15) mit dem Mündungsrand (8) der Einfüllöffnung (7) zusammenfällt, **dadurch gekennzeichnet, daß** nach dem Befüllen der jeweiligen Ampulle (1) mit ihrer Soll-Füllmenge (V) in mehreren aufeinanderfolgenden, gleichartigen Zyklen die Einfüllöffnung (7) zunächst gasdicht verschlossen wird, daß anschließend der Stopfen (5) mittels einer Eindrückeinrichtung (21) mit einer vorgegebenen Kraft (F) in Richtung der Einfüllöffnung (7) verschoben wird, daß danach die auf den Stopfen (5) von der Eindrückeinrichtung (21) ausgeübte Kraft (F) reduziert wird und daß zuletzt die Einfüllöffnung (7) derart entlastet wird, daß die durch den Eindrückvorgang des Stopfens (5) in dem Kopfraum (13) der Ampulle (1) komprimierte Luft aus der Ampulle (1) entweicht.

2. Verfahren nach Anspruch 1, **dadurch gekennzeichnet, daß** die Anzahl der Zyklen unter Berücksichtigung der vorgegebenen Kraft (F) als Festwert in einer Steuereinrichtung abgelegt ist.

3. Verfahren nach Anspruch 1, **dadurch gekennzeichnet, daß** sich die Anzahl der Zyklen in Abhängigkeit des Verschiebewegs (s) des Stopfens (5) ergibt, derart, daß beim Unterschreiten eines vorgegebenen Verschiebewegs s(min) der Vorgang des Stopfenverschiebens abgeschlossen ist.

4. Verfahren zum Befüllen von im wesentlichen zylinderförmigen Ampullen (1), welche zwei Öffnungen (4, 7) aufweisen, eine untere Öffnung (4), in der ein kolbenartiger Stopfen (5) verschiebbar angeordnet ist, und eine obere Einfüllöffnung (7) für ein flüssiges Füllgut (6), die von einer Abdeckkappe (11), gegebenenfalls unter Zwischenlage eines Dichtelements (9), nach dem Befüllen verschlossen wird, wobei der Füllpegel (15) des Füllgutes (6) zur Vermeidung von Einschluss von Luft in den Ampullen (1) bis in Höhe des Mündungsrandes (8) der oberen Einfüllöffnung (7) ragt, wobei der Stopfen (5) zunächst in einer Position angeordnet ist, in der der Füllpegel (15) des Füllgutes (6) unter Berücksichtigung einer Soll-Füllmenge (V) unterhalb des Mündungsrandes (8) der Einfüllöffnung (7) verläuft, wobei anschließend die Soll-Füllmenge (V) in die jeweilige Ampulle (1) eingebracht wird und wobei zuletzt der Stopfen (5) in eine Position verschoben wird, in der der Füllpegel (15) mit dem Mündungsrand (8) der Einfüllöffnung (7) zusammenfällt, **dadurch gekennzeichnet, daß** zunächst die Einfüllöffnung (7) der Ampulle (1) dicht verschlossen wird, daß anschließend der Stopfen (5) mittels einer Eindrückeinrichtung (21) in Richtung der Einfüllöffnung (7) um eine bestimmte Weglänge (s(ref)) verschoben wird, wobei gleichzeitig über den Stopfeneindrückweg (s) die Stopfeneindrückkraft (F(ref)) erfaßt wird, daß aus dem Stopfeneindrückwegs (s(ref)) und der Stopfeneindrückkraft (F(ref)) ein füllgutfreies Kopfraumvolumen (K) in der Ampulle (1) berechnet wird, daß danach die Einfüllöffnung (7) zum Luftaustritt freigegeben wird und daß zuletzt der Stopfen (5) um eine dem füllgutfreien Kopfvolumenvolumen (K) entsprechende Weglänge (s) weiter in Richtung der Einfüllöffnung (7) eingeschoben wird.

5. Verfahren zum Befüllen von im wesentlichen zylinderförmigen Ampullen (1), welche zwei Öffnungen (4, 7) aufweisen, eine untere Öffnung (4), in der ein kolbenartiger Stopfen (5) verschiebbar angeordnet ist, und eine obere Einfüllöffnung (7) für ein flüssiges Füllgut (6), die von einer Abdeckkappe (11), gegebenenfalls unter Zwischenlage eines Dichtelements (9), nach dem Befüllen verschlossen wird, wobei der Füllpegel (15) des Füllgutes (6) zur Vermeidung von Einschluss von Luft in den Ampullen (1) bis in Höhe des Mündungsrandes (8) der oberen Einfüllöffnung (7) ragt, wobei der Stopfen (5) zunächst in einer Position angeordnet ist, in der der Füllpegel (15) des Füllgutes (6) unter Berücksichtigung einer Soll-Füllmenge (V) unterhalb des Mündungsrandes (8) der Einfüllöffnung (7) verläuft, wobei anschließend die Soll-Füllmenge (V) in die jeweilige Ampulle (1) eingebracht wird und wobei zuletzt der Stopfen (5) in eine Position verschoben wird, in der der Füllpegel (15) mit dem Mündungsrand (8) der Einfüllöffnung (7) zusammenfällt, **dadurch gekennzeichnet, daß** zunächst die Einfüllöffnung (7) der Ampulle (1) dicht verschlossen wird, daß anschließend der Stopfen (5) mittels einer Eindrückeinrichtung (21) in Richtung der Einfüllöffnung (7) um eine bestimmte Weglänge (s(ref)) verschoben wird, wobei gleichzeitig über den Stopfeneindrückweg (s) der im füllgutfreien Kopfraum (13) der Ampulle (1) herrschende Druckverlauf mittels eines Drucksensors (27) erfaßt wird, daß aus dem Verlauf des Stopfeneindrückwegs (s) und des Druckverlaufs ein füllgutfreies Kopfraumvolumen (K) in der Ampulle (1) bestimmt wird, daß danach die Einfüllöffnung (7) zum Luftaustritt freigegeben wird und daß zuletzt der Stopfen (5) in der Ampulle (1) um eine dem füllgutfreien Kopfraum (13) entsprechende Weglänge (s) weiter in Richtung der Einfüllöffnung (7) eingeschoben wird.

6. Verfahren zum Befüllen von im wesentlichen zylinderförmigen Ampullen (1), welche zwei Öffnungen (4, 7) aufweisen, eine untere Öffnung (4), in der ein kolbenartiger Stopfen (5) verschiebbar angeordnet ist, und eine obere Einfüllöffnung (7) für ein flüssiges Füllgut (6), die von einer Abdeckkappe (11), gegebenenfalls unter Zwischenlage eines Dichtelements (9), nach dem Befüllen verschlossen wird, wobei der Füllpegel (15) des Füllgutes (6) zur Vermeidung von Einschluss von Luft in den Ampullen (1) bis in Höhe des Mündungsrandes (8) der oberen Einfüllöffnung (7) ragt, wobei der Stopfen (5) zunächst in einer Position angeordnet ist, in der der Füllpegel (15) des Füllgutes (6) unter Berücksichtigung einer soll-Füllmenge (V) unterhalb des Mündungsrandes (8) der Einfüllöffnung (7) verläuft, wobei anschließend die Soll-Füllmenge (V) in die jeweilige Ampulle (1) eingebracht wird und wobei zuletzt der Stopfen (5) in eine Position verschoben wird, in der der Füllpegel (15) mit dem Mündungsrand (8) der Einfüllöffnung (7) zusammenfällt, **dadurch gekennzeichnet, daß** zunächst mittels einer Vakuumeinrichtung (30) der füllgutfreie Kopfraum (13) der Ampulle (1) evakuiert wird, daß anschließend die Einfüllöffnung (7) dicht verschlossen wird und daß zuletzt der Stopfen (5) mittels einer Eindrückeinrichtung (21) so weit in Richtung der Einfüllöffnung (7) verschoben wird, bis die auf die Eindrückeinrichtung (21) vom Stopfen (5) erzeugte Reaktionskraft (F(reak)) einen vorgegebenen Wert übersteigt.

7. Verfahren zum Befüllen von im wesentlichen zylinderförmigen Ampullen (1), welche zwei Öffnungen (4, 7) aufweisen, eine untere Öffnung (4), in der ein kolbenartiger Stopfen (5) verschiebbar angeordnet ist, und eine obere Einfüllöffnung (7) für ein flüssiges Füllgut (6), die von einer Abdeckkappe (11), gegebenenfalls unter Zwischenlage eines Dichtelements (9), nach dem Befüllen verschlossen wird, wobei der Füllpegel (15) des Füllgutes (6) zur Vermeidung von Einschluss von Luft in den Ampullen (1) bis in Höhe des Mündungsrandes (8) der oberen Einfüllöffnung (7) ragt, wobei der Stopfen (5) zunächst in einer Position angeordnet ist, in der der Füllpegel (15) des Füllgutes (6) unter Berücksichtigung einer Soll-Füllmenge (V) unterhalb des Mündungsrandes (8) der Einfüllöffnung (7) verläuft, wobei anschließend die Soll-Füllmenge (V) in die jeweilige Ampulle (1) eingebracht wird und wobei zuletzt der Stopfen (5) in eine Position verschoben wird, in der der Füllpegel (15) mit dem Mündungsrand (8) der Einfüllöffnung (7) zusammentällt, **dadurch gekennzeichnet, daß** der Füllpegel (15) der Soll-Füllmenge (V) mittels einer vorzugsweise optischen Kontrolleinrichtung (35) erfaßt wird, daß aus der erfaßten Position des Füllpegels (15) ein füllgutfreies Kopfraumvolumen (K) in der Ampulle (1) berechnet wird und daß zuletzt der Stopfen (5) um eine bestimmte, dem berechneten füllgutfreien Kopfraumvolumen (K) entsprechende Weglänge (s) mittels einer Eindrückeinrichtung (21) in Richtung der Einfüllöffnung (7) verschoben wird.

8. Verfahren nach einem der Ansprüche 1 bis 4 oder 6, **dadurch gekennzeichnet, daß** die Einfüllöffnung (7) von der Abdeckkappe (11), gegebenenfalls unter Zwischenlage des Dichtelements (9) abgedichtet wird und daß zuletzt die Einfüllöffnung (7) mittels der Abdeckkappe (11) dicht verschlossen wird.

## Claims

1. Method for filling essentially cylindrical ampoules (1) which have two openings (4, 7), a lower opening (4), in which a plunger-like stopper (5) is displaceably arranged, and an upper pour-in opening (7) for a liquid filling material (6), which opening is closed after the filling by a covering cap (11), if appropriate with the interposition of a sealing element (9), the filling level (15) of the filling material (6) protruding to the level of the mouth edge (8) of the upper pour-in opening (7) in order to avoid air being trapped in the ampoules (1), the stopper (5) initially being arranged in a position in which the filling level (15) of the filling material (6), taking a desired filling quantity (V) into consideration, runs below the mouth edge (8) of the pour-in opening (7), the desired filling quantity (V) then being inserted into the particular ampoule (1), and finally the stopper (5) being displaced into a position in which the filling level (15) coincides with the mouth edge (8) of the pour-in opening (7), **characterized in that**, after the filling of the particular ampoule (1) with its desired filling quantity (V) in a plurality of consecutive, identical cycles, the pour-in opening (7) is initially closed in a gas-tight manner, **in that** the stopper (5) is then displaced by means of a press-in device (21) at a predetermined force (F) in the direction of the pour-in opening (7), **in that** the force (F) exerted on the stopper (5) by the press-in device (21) is then reduced, and **in that** finally the pour-in opening (7) is relieved of load in such a manner that the air compressed in the head space (13) of the ampoule (1) by the press-in process of the stopper (5) escapes from the ampoule (1).

2. Method according to Claim 1, **characterized in that** the number of cycles is deposited, with the predetermined force (F) taken into consideration, as a fixed value in a control device.

3. Method according to Claim 1, **characterized in that** the number of cycles arises as a function of the displacement travel (s) of the stopper (5) in such a manner that, if the displacement travel s(min) falls short of a predetermined value, the process of the displacement of the stopper is terminated.

4. Method for filling essentially cylindrical ampoules (1) which have two openings (4, 7), a lower opening (4), in which a plunger-like stopper (5) is displaceably arranged, and an upper pour-in opening (7) for a liquid filling material (6), which opening is closed after the filling by a covering cap (11), if appropriate with the interposition of a sealing element (9), the filling level (15) of the filling material (6) protruding to the level of the mouth edge (8) of the upper pour-in opening (7) in order to avoid air being trapped in the ampoules (1), the stopper (5) initially being arranged in a position, in which the filling level (15) of the filling material (6), taking a desired filling quantity (V) into consideration, runs below the mouth edge (8) of the pour-in opening (7), the desired filling quantity (V) then being inserted into the particular ampoule (1), and finally the stopper (5) being displaced into a position in which the filling level (15) coincides with the mouth edge (8) of the pour-in opening (7), **characterized in that**, the pour-in opening (7) of the ampoule (1) is initially tightly closed, **in that** the stopper (5) is then displaced by means of a press-in device (21) in the direction of the pour-in opening (7) by a certain travel length (s(ref)), the stopper press-in force (F(ref)) being detected at the same time via the stopper press-in travel (s), **in that** a filling-material-free head-space volume (K) in the ampoule (1) is calculated from the stopper press-in travel (s(ref)) and the stopper press-in force (F(ref)), **in that** the pour-in opening (7) is then opened up in order to let the air out, and **in that** finally the stopper (5) is pushed in further in the direction of the pour-in opening (7) by a travel length (s) corresponding to the filling-material-free head-space volume (K).

5. Method for filling essentially cylindrical ampoules (1) which have two openings (4, 7), a lower opening (4), in which a plunger-like stopper (5) is displaceably arranged, and an upper pour-in opening (7) for a liquid filling material (6), which opening is closed after the filling by a covering cap (11), if appropriate with the interposition of a sealing element (9), the filling level (15) of the filling material (6) protruding to the level of the mouth edge (8) of the upper pour-in opening (7) in order to avoid air being trapped in the ampoules (1), the stopper (5) initially being arranged in a position in which the filling level (15) of the filling material (6), taking a desired filling quantity (V) into consideration, runs below the mouth edge (8) of the pour-in opening (7), the desired filling quantity (V) then being inserted into the particular ampoule (1), and finally the stopper (5) being displaced into a position in which the filling level (15) coincides with the mouth edge (8) of the pour-in opening (7), **characterized in that** the pour-in opening (7) of the ampoule (1) is initially tightly closed, **in that** the stopper (5) is then displaced by means of a press-in device (21) in the direction of the pour-in opening (7) by a certain travel length (s(ref)), the pressure profile prevailing in the filling-material-free head space (13) of the ampoule (1) being detected at the same time via the stopper press-in travel (s) by means of a pressure sensor (27), **in that** a filling-material-free head-space volume (K) in the ampoule (1) is determined from the profile of the stopper press-in travel (s) and the pressure profile, **in that** the pour-in opening (7) is then opened up to let the air out, and **in that** finally the stopper (5) is pushed further in in the ampoule (1) in the direction of the pour-in opening (7) by a travel length (s) corresponding to the filling-material-free head space (13).

6. Method for filling essentially cylindrical ampoules (1) which have two openings (4, 7), a lower opening (4), in which a plunger-like stopper (5) is displaceably arranged, and an upper pour-in opening (7) for a liquid filling material (6), which opening is closed after the filling by a covering cap (11), if appropriate with the interposition of a sealing element (9), the filling level (15) of the filling material (6) protruding to the level of the mouth edge (8) of the upper pour-in opening (7) in order to avoid air being trapped in the ampoules (1), the stopper (5) initially being arranged in a position in which the filling level (15) of the filling material (6), taking a desired filling quantity (V) into consideration, runs below the mouth edge (8) of the pour-in opening (7), the desired filling quantity (V) then being inserted into the particular ampoule (1), and finally the stopper (5) being displaced into a position in which the filling level (15) coincides with the mouth edge (8) of the pour-in opening (7), **characterized in that** the filling-material-free head space (13) of the ampoule (1) is first of all evacuated by means of a vacuum device (30), **in that** the pour-in opening (7) is then tightly closed, and **in that** finally the stopper (5) is displaced by means of a press-in device (21) in the direction of the pour-in opening (7) until the reaction force (F(reac)) produced on the press-in device (21) by the stopper (5) exceeds a predetermined value.

7. Method for filling essentially cylindrical ampoules (1) which have two openings (4, 7), a lower opening (4), in which a plunger-like stopper (5) is displaceably arranged, and an upper pour-in opening (7) for a liquid filling material (6), which opening is closed after the filling by a covering cap (11), if appropriate with the interposition of a sealing element (9), the filling level (15) of the filling material (6) protruding to the level of the mouth edge (8) of the upper pour-in opening (7) in order to avoid air being trapped in the ampoules (1), the stopper (5) initially being arranged in a position in which the filling level (15) of the filling material (6), taking a desired filling quantity (V) into consideration, runs below the mouth edge (8) of the pour-in opening (7), the desired filling quantity (V) then being inserted into the particular ampoule (1), and finally the stopper (5) being displaced into a position in which the filling level (15) coincides with the mouth edge (8) of the pour-in opening (7), **characterized in that** the filling level (15) of the desired filling quantity (V) is detected by means of a preferably optical checking device (35), **in that** a filling-material-free head-space volume (K) in the ampoule (1) is calculated from the detected position of the filling level (15) and **in that** finally the stopper (5) is displaced by means of a press-in device (21) in the direction of the pour-in opening (7) by a certain travel length (s) corresponding to the calculated, filling-material-free head-space volume (K).

8. Method according to one of Claims 1 to 4 or 6, **characterized in that** the pour-in opening (7) is sealed by the covering cap (11), if appropriate with the interposition of the sealing element (9), and **in that** finally the pour-in opening (7) is tightly closed by means of the covering cap (11).

## Revendications

1. Procédé pour remplir des ampoules (1) pratiquement cylindriques munies de deux ouvertures (4, 7), d'une ouverture inférieure (4) dans laquelle peut se déplacer un bouchon (5) en forme de piston et d'une ouverture de remplissage (7) supérieure, destinée à un produit de remplissage (6) liquide, qu'on ferme après le remplissage à l'aide d'un capuchon (11) en intercalant éventuellement un élément d'étanchéité (9),
selon lequel le niveau de remplissage (15) du produit de remplissage (6) atteint le niveau du bord d'orifice (8) de l'ouverture de remplissage supérieure (7) afin d'éviter qu'il n'y ait des inclusions d'air dans les ampoules (1), le bouchon (5) est d'abord placé dans une position où le niveau de remplissage (15) du produit de remplissage (6) se trouve au-dessous du bord d'orifice (8) de l'ouverture de remplissage (7) en tenant compte d'une quantité de consigne de remplissage (V) puis la quantité théorique de remplissage (V) est introduite dans chaque ampoule (1) et
finalement le bouchon (5) est poussé dans une position où le niveau de remplissage (15) coïncide avec le bord d'orifice (8) de l'ouverture de remplissage (7),
**caractérisé en ce que**
après chaque remplissage d'ampoule (1) par sa quantité de consigne de remplissage (V), au cours de plusieurs cycles successifs et similaires, d'abord l'ouverture de remplissage (7) est fermée de façon étanche au gaz, puis le bouchon (5) est poussé en direction de l'ouverture de remplissage (7) à l'aide d'une installation d'insertion (21) selon une force (F) prédéfinie, puis la force (F) exercée par l'installation d'insertion (21) sur le bouchon (5) est réduite, et
enfin l'ouverture de remplissage (7) est déchargée de telle manière que l'air, comprimé par la pénétration du bouchon (5) dans l'espace de tête (13) de l'ampoule (1), s'échappe de l'ampoule (1).

2. Procédé selon la revendication 1,
**caractérisé en ce que**
le nombre de cycles est enregistré en tant que valeur fixe dans une installation de commande en tenant compte de la force (F) prédéfinie.

3. Procédé selon la revendication 1,
**caractérisé en ce que**
on obtient le nombre de cycles en fonction de la course (s) du bouchon (5) de telle manière que le déplacement du bouchon cesse avant qu'on n'atteigne une course s(min) prédéfinie.

4. Procédé pour remplir des ampoules (1) pratiquement cylindriques munies de deux ouvertures (4, 7), d'une ouverture inférieure (4) dans laquelle peut se déplacer un bouchon (5) en forme de piston et d'une ouverture de remplissage (7) supérieure, destinée à un produit de remplissage (6) liquide, qu'on ferme après le remplissage à l'aide d'un capuchon (11) en intercalant éventuellement un élément d'étanchéité (9),
selon lequel le niveau de remplissage (15) du produit de remplissage (6) atteint le niveau du bord d'orifice (8) de l'ouverture de remplissage supérieure (7) afin d'éviter qu'il n'y ait des inclusions d'air dans les ampoules (1), le bouchon (5) est d'abord placé dans une position où le niveau de remplissage (15) du produit de remplissage (6) se trouve au-dessous du bord d'orifice (8) de l'ouverture de remplissage (7) en tenant compte d'une quantité de consigne de remplissage (V) puis la quantité théorique de remplissage M est introduite dans chaque ampoule (1) et
finalement le bouchon (5) est poussé dans une position où le niveau de remplissage (15) coïncide avec le bord d'orifice (8) de l'ouverture de remplissage (7),
**caractérisé en ce que**
d'abord l'ouverture de remplissage (7) de l'ampoule (1) est hermétiquement fermée, puis le bouchon (5) est poussé sur une course (s(ref)) prédéfinie en direction de l'ouverture de remplissage (7) à l'aide d'une installation d'insertion (21), en même temps la force de pénétration du bouchon (F(ref)) est détectée sur la course de pénétration du bouchon (s), on calcule un volume d'espace de tête (K) sans produit de remplissage dans l'ampoule (1) à partir de la course de pénétration du bouchon (s(ref)) et de la force de pénétration du bouchon (F(ref)) puis l'ouverture de remplissage (7) est libérée pour faire sortir l'air et
finalement le bouchon (5) est introduit plus loin en direction de l'ouverture de remplissage (7) selon une course (s) qui correspond au volume d'espace de tête (K) sans produit de remplissage.

5. Procédé pour remplir des ampoules (1) pratiquement cylindriques munies de deux ouvertures (4, 7), d'une ouverture inférieure (4) dans laquelle peut se déplacer un bouchon (5) en forme de piston et d'une ouverture de remplissage (7) supérieure, destinée à un produit de remplissage (6) liquide, qu'on ferme après le remplissage à l'aide d'un capuchon (11) en intercalant éventuellement un élément d'étanchéité (9), selon lequel le niveau de remplissage (15) du produit de remplissage (6) atteint le niveau du bord d'orifice (8) de l'ouverture de remplissage supérieure (7) afin d'éviter qu'il n'y ait des inclusions d'air dans les ampoules (1), le bouchon (5) est d'abord placé dans une position où le niveau de remplissage (15) du produit de remplissage (6) se trouve au-dessous du bord d'orifice (8) de l'ouverture de remplissage (7) en tenant compte d'une quantité de consigne de remplissage (V) puis la quantité de consigne de remplissage (V) est introduite dans chaque ampoule (1) et
finalement le bouchon (5) est poussé dans une position où le niveau de remplissage (15) coïncide avec le bord d'orifice (8) de l'ouverture de remplissage (7),
**caractérisé en ce que**
d'abord l'ouverture de remplissage (7) de l'ampoule (1) est hermétiquement fermée, puis le bouchon (5) est poussé sur une course (s(ref)) prédéfinie en direction de l'ouverture de remplissage (7) à l'aide d'une installation d'insertion (21), en même temps le tracé de la pression qui règne dans l'espace de tête (13) - de l'ampoule (1) - sans produit de remplissage est détecté sur la course de pénétration du bouchon (s) à l'aide d'un capteur de pression (27), un volume d'espace de tête (K) - sans produit de remplissage - dans l'ampoule (1) est déterminé à partir du tracé de la course de pénétration du bouchon (s) et du tracé de la pression puis l'ouverture de remplissage (7) est libérée pour faire sortir l'air, et
finalement le bouchon (5) est introduit dans l'ampoule (1) plus avant en direction de l'ouverture de remplissage (7) selon une course (s) qui correspond à l'espace de tête (13) sans produit de remplissage.

6. Procédé pour remplir des ampoules (1) pratiquement cylindriques munies de deux ouvertures (4, 7), d'une ouverture inférieure (4) dans laquelle peut se déplacer un bouchon (5) en forme de piston et d'une ouverture de remplissage (7) supérieure, destinée à un produit de remplissage (6) liquide, qu'on ferme après le remplissage à l'aide d'un capuchon (11) en intercalant éventuellement un élément d'étanchéité (9), selon lequel le niveau de remplissage (15) du produit de remplissage (6) atteint le niveau du bord d'orifice (8) de l'ouverture de remplissage supérieure (7) afin d'éviter qu'il n'y ait des inclusions d'air dans les ampoules (1), le bouchon (5) est d'abord placé dans une position où le niveau de remplissage (15) du produit de remplissage (6) se trouve au-dessous du bord d'orifice (8) de l'ouverture de remplissage (7) en tenant compte d'une quantité d'insertion de remplissage (V) puis la quantité théorique de remplissage (V) est introduite dans chaque ampoule (1) et
finalement le bouchon (5) est poussé dans une position où le niveau de remplissage (15) coïncide avec le bord d'orifice (8) de l'ouverture de remplissage (7),
**caractérisé en ce que**
d'abord l'espace de tête (13) - sans produit de remplissage - de l'ampoule (1) est vidé à l'aide d'une installation de vide (30), puis l'ouverture de remplissage (7) est hermétiquement fermée, et
finalement le bouchon (5) est poussé à l'aide d'une installation d'insertion (21) en direction de l'ouverture de remplissage (7) jusqu'à ce que la force de réaction (F(reak)) produite par le bouchon (5) sur l'installation d'insertion (21) dépasse une valeur prédéfinie.

7. Procédé pour remplir des ampoules (1) pratiquement cylindriques munies de deux ouvertures (4, 7), d'une ouverture inférieure (4) dans laquelle peut se déplacer un bouchon (5) en forme de piston et d'une ouverture de remplissage (7) supérieure, destinée à un produit de remplissage (6) liquide, qu'on ferme après le remplissage à l'aide d'un capuchon (11) en intercalant éventuellement un élément d'étanchéité (9), selon lequel le niveau de remplissage (15) du produit de remplissage (6) atteint le niveau du bord d'orifice (8) de l'ouverture de remplissage supérieure (7) afin d'éviter qu'il n'y ait des inclusions d'air dans les ampoules (1), le bouchon (5) est d'abord placé dans une position où le niveau de remplissage (15) du produit de remplissage (6) se trouve au-dessous du bord d'orifice (8) de l'ouverture de remplissage (7) en tenant compte d'une quantité de consigne de remplissage (V) puis la quantité de consigne de remplissage (V) est introduite dans chaque ampoule (1) et
finalement le bouchon (5) est poussé dans une position où le niveau de remplissage (15) coïncide avec le bord d'orifice (8) de l'ouverture de remplissage (7),
**caractérisé en ce que**
le niveau de remplissage (15) de la quantité de consigne de remplissage (V) est détecté à l'aide d'une installation de contrôle (35) de préférence optique, un volume d'espace de tête (K) - sans produit de remplissage - dans l'ampoule (1) est calculé à partir de la position détectée du niveau de remplissage (15) et finalement le bouchon (5) est poussé à l'aide d'une installation de consigne (21) en direction de l'ouverture de remplissage (7) selon une course (s) définie qui correspond au volume d'espace de tête (K) calculé sans produit de remplissage.

8. Procédé selon l'une des revendications 1 à 4 ou 6,
**caractérisé en ce que**
l'ouverture de remplissage (7) est rendue étanche par le capuchon (11), en intercalant éventuellement l'élément d'étanchéité (9), et
finalement l'ouverture de remplissage (7) est hermétiquement fermée à l'aide du capuchon (11).
